# EUROPEAN PATENT APPLICATION

(11) **EP 4 696 338 A1**
(43) Date of publication of application: **18.02.2026**
(21) Application number: 24807545.9
(22) Date of filing: 14.05.2024
(51) Int. Cl.: A61L 27/26, A61L 27/18

(54) **FILLER COMPOSITION FOR CHONDROCYTE DIFFERENTIATION ABILITY AND COLLAGEN ACTIVITY, AND PREPARATION METHOD THEREFOR**

(30) Priority: 15.05.2023 KR 20230062184
(71) Applicant: Ultra V Co., Ltd., Incheon 21069 (KR)
(72) Inventor: KWON, Han Jin, Seoul 06092 (KR); HAM, Jung Ryul, Paju-si, Gyeonggi-do 10851 (KR); LEE, Won Ku, Gimpo-si, Gyeonggi-do 10077 (KR)
(74) Representative: BCKIP Part mbB
(86) International application number: PCT/KR2024/006597
(87) International publication number: WO 2024/237674

(57) **Abstract**

The present invention relates to a filler composition for chondrocyte differentiation ability and collagen activity, and a preparation method therefor. The composition comprises a mixture of PLGA microparticles and HA and PDRN microparticles, and can be usefully used as a filler composition by stably inducing cell adsorption, increasing collagen production, activating chondrocyte regeneration, and confirming a tissue repair effect.

## Description

### THECHNICAL FIELD

The present invention relates to a filler composition for chondrocyte differentiation ability and collagen activity, and a preparation method therefor.

### BACKGROUND OF THE INVENTION

The customer base for the anti-aging industry, encompassing functional cosmetics for skin, fillers, botulinum toxin, and beauty services, is expanding to the general public, including the middle class. Interest in human cell activation is growing due to trends in life extension, wellness, and advances in biotechnology.

Within the anti-aging industry, products and services related to chondrocyte activation can be divided into consumer goods (such as cosmetics), medical products, and service sectors. The medical field, in particular, is growing as various products related to pharmaceuticals and biomaterials, such as botulinum toxin and hyaluronic acid fillers, are being developed.

Botulinum toxin, originally utilized for treating strabismus because of its muscle-relaxing properties, approximately 90% of clinical applications in Korea are for cosmetic purposes. The filler market is also growing rapidly with the commercialization of safe, rapidly bioabsorbable pharmaceutical materials like hyaluronic acid and collagen.

Furthermore, PDRN promotes the regeneration and growth of bone tissue (Guizzardi et al., Micron, 38, 2007, 722-728) and improves damaged skin repair and wound recovery in diabetic animal models. This occurs because it promotes the expression of VEGF (Vascular Endothelial Growth Factor) through key factors regulating angiogenesis. PDRN is also effective for skin injuries where regeneration and angiogenesis are difficult, such as thermal injury. Administration of PDRN^{®} to deep-dermal second-degree burn injuries accelerated re-epithelialization, shortening the time required for final wound closure (Bitto et al., Crit. Care. Med., 36, 2008a, 1594-1602).

Meanwhile, among cosmetic fillers, microparticles composed of hydrophobic biodegradable polymers are characterized by complete degradation within the body through hydrolysis, with the degradation products excreted in urine. They offer the advantage of being adjustable in duration, ranging from as long as 4 years to as short as 6 months, depending on the type and molecular weight of the polymer.

Various filler compositions composed of hydrophobic biodegradable pol ymers such as Poly-L-Lactic acid (PLLA), Poly-ε-caprolactone (PCL), PLGA (poly(lactic-co-glycolic acid)), and polydioxanone (PDO) are being proposed.

However, PDO has the disadvantage of being difficult to maintain long-term due to its short degradation period. While PLGA, PLLA, and PCL maintain a degradation period of over one year, their high hydrophobicity is reported to cause side effects such as granuloma formation.

In particular, after the injection of hydrophobic biodegradable polymer microparticles into the human body, they may induce an effect of collagen regeneration through a foreign body reaction However, this process has the disadvantage that cell adhesion to the surface of the hydrophobic biodegradable polymer microparticles is difficult, leading to slow or inadequate collagen regeneration.

Against this background, the inventors conducted research to develop a filler that facilitates cell adsorption and possesses a retention period of over one year in vivo. As a result, they completed the present invention by manufacturing a filler composition hydrolyzed from a mixture of PLGA microparticles, HA, and PDRN, and confirming that this composition increases cell adsorption, possesses a retention period of over one year, and promotes skin regeneration.

### PRIOR ART LITERATURE

### PATENT LITERATURE

(Patent Reference 0001) Republic of Korea Registered Patent No. KR10-1926751

### DETAILED DESCRIPTION OF THE INVENTION

### TECHNICAL PROBLEM

An objective of the present invention is to provide a method for preparing a filler composition for plastic surgery or chondrocyte regeneration comprising the steps of: i) preparing PLGA microparticles composed of PLGA; ii) providing HA and PDRN microparticles; iii) preparing a mixture by mixing the PLGA microparticles with the HA and PDRN microparticles; iv) hydrolyzing the mixture; v) preparing a dispersion by dispersing the hydrolyzed mixture in a solution containing a dispersant; and vi) preparing a complex comprising the PLGA microparticles, the HA and PDRN microparticles by freeze-drying the dispersion.

Another objective of the present invention is to provide a filler composition for plastic surgery or chondrocyte regeneration prepared by the method.

Yet another objective of the present invention is to provide an injectable formulation for plastic surgery or chondrocyte regeneration, comprising the filler composition.

### TECHNICAL SOLUTION

The advantages and features of the present invention, and the methods for achieving them, will become clear upon reference to the detailed description of the embodiments disclosed herein, together with the accompanying figures. However, the present invention is not limited to the embodiments disclosed below and may be implemented in various different forms. These embodiments are provided solely to make the disclosure of the present invention complete and to fully inform a person skilled in the art to which the present invention pertains of the scope of the invention. The scope of the invention is defined solely by the scope of the claims.

The terms used herein are for illustrating the embodiments and are not intended to limit the invention. In this specification, the singular form includes the plural form unless specifically stated otherwise in the context. The terms "comprises" and/or "comprising" as used herein do not exclude the presence or addition of at least one other components besides those mentioned. Throughout the specification, the same figure numerals denote the same components, and "and/or" includes each of the mentioned components and any and all combinations thereof. Although "first," "second," etc., are used to describe various components, these components are not limited by these terms. These terms are used merely to distinguish one component from another. Therefore, the first component mentioned below may also be the second component within the technical concept of the present invention.

Unless otherwise defined, all terms used herein (including technical and scientific terms) may be used in a meaning that can be commonly understood by a person skilled in the art to which the present invention belongs. Furthermore, terms defined in commonly used dictionaries shall not be interpreted ideally or excessively unless explicitly defined otherwise.

The present invention provides a method for preparing a filler composition for plastic surgery or chondrocyte regeneration comprising the steps of: i) preparing PLGA microparticles composed of PLGA; ii) providing HA and PDRN microparticles; iii) preparing a mixture by mixing the PLGA microparticles with the HA and PDRN microparticles; iv) hydrolyzing the mixture; v) preparing a dispersion by dispersing the hydrolyzed mixture in a solution containing a dispersant; and vi) preparing a complex comprising the PLGA microparticles, the HA and PDRN microparticles by freeze-drying the dispersion.

The PLGA of the present invention is a poly(lactic-co-glycolic acid) copolymer, which is a biodegradable and biocompatible polymer. The PLGA can yield polymers with different forms depending on the ratio of lactide and glycolide, and the crystallinity, glass transition temperature, and hydrolysis time of the PLGA can vary according to this ratio. The PLGA is non-toxic, metabolizable, and excretable, and possesses excellent physical properties, making it suitable for use as a therapeutic device such as an implant or surgical device.

The HA of the present invention is hyaluronic acid, a polymer compound defined as a glycosaminoglycan possessing an unbranched polysaccharide chain formed by the condensation of thousands of disaccharide units, exhibiting high solubility in water. By forming cross-linked hyaluronic acid using specific cross-linking agents and appropriate operating conditions (temperature, pH, etc.), a molecular framework can be created to maintain the shape and tone of epidermal tissue.

The PDRN of the present invention is a polydeoxyribonucleotide, a compound comprising a DNA complex, namely a deoxyribonucleotide polymer, which can be used as an active agent necessary for cell regeneration without side effects and can promote cell differentiation.

In the method for preparing a filler composition of the present invention, wherein the weight ratio of the PLGA microparticles based on the total weight of the mixture may be 70 to 90 wt% in the step iii), more specifically may be 75 to 90 %, but is not limited thereto.

The weight ratio of the HA based on the total weight of the mixture may be 1 to 20 wt% in the step iii), and more specifically may be 1 to 5%, but is not limited thereto.

The weight ratio of the PDRN microparticles based on the total weight of the mixture may be from 0.1 to 5 wt% in the step iii), and more specifically may be from 0.01 to 1%, but is not limited thereto.

The weight ratio of the HA and PDRN microparticles based on the total weight of the mixture may be from 5 to 25 wt% in the step iii), but is not limited thereto.

The weight ratio of the PLGA microparticles in the total weight of the mixture may be 70 to 90 wt%, outside this range, cell adhesion becomes unstable, delaying collagen production and shortening the collagen retention period, making it difficult to achieve long-term tissue repair effects.

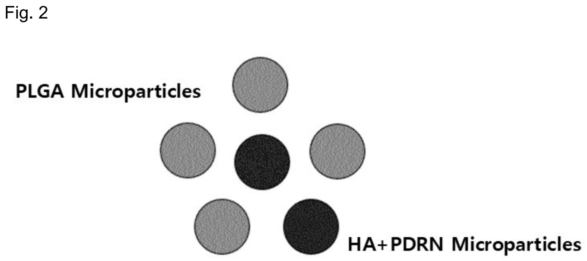

For example, a filler composition for plastic surgery comprising a mixture of PLGA microparticles and HA and PDRN microparticles, wherein the PLGA microparticles constitute 75% by weight and the HA and PDRN microparticles constitute up to 25%, allows sufficient HA and PDRN microparticles to be present around the PLGA microparticles(Fig. 2).

The dispersant of the present invention is used to disperse bioactive substances, and either a low molecular weight dispersant or a high molecular weight dispersant polymer may be applied. The low molecular weight dispersant refers to a compound with a weight average molecular weight of less than 15,000 MW, and the high molecular weight dispersant polymer refers to a compound containing covalent bonds between one or more monomers and having a weight average molecular weight of 15,000 MW or more. As low molecular weight dispersants, those permitted for pharmaceutical compounds, functional food compounds, functional cosmetic compounds, etc., are not particularly restricted. Specifically, they may be sugars, cyclodextrins, amino acids, organic acids, or other components. They may be used alone or in combination with two or more types.

The dispersant of the present invention may be selected from the group consisting of cells, growth factors, peptides, amino acids, proteins, carbohydrates, natural polymers, and combinations thereof, but is not limited thereto.

The cells and growth factors of the present invention may be at least one cells and growth factors selected from the group consisting of mesenchymal stem cells, induced pluripotent stem cells (iPSCs), fibroblast growth factor (FGF), epidermal growth factor (EGF), keratinocyte growth factor (KGF), transforming growth factor alpha (TGF-α), transforming growth factor beta (TGF-β), granulocyte-colony stimulating factor (GCSF), insulin-like growth factor (IGF), vascular endothelial growth factor (VEGF), hepatocyte growth factor (HGF), platelet-derived growth factor-BB (PDGF-BB), brain-derived neurotrophic factor (BDNF) and glial cell-derived neurotrophic factor (GDNF).

The peptides, amino acids, and proteins of the present invention may be Alanine, Arginine, Valine, Asparagine, Aspartic acid, Cysteine, Glutamine, Glutamic acid, Glycine, Histidine, Isoleucine, Lysine, Methionine, Phenylalanine, Proline, Serine, Threonine, Tryptophan, Tyrosine, or combinations thereof.

The sugar of the present invention may be Glucose, Mannose, Idose, galactose, fucose, ribose, xylose, lactose, sucrose, maltose, trehalose, turanose, Raffinose, Maltotriose, Acarbose, water-soluble cellulose, Synthetic Cellulose, Sugar Alcohol, glycerin, sorbitol, Lactitol, mannitol, Ksylitoli, Erythritol, polyol, derivatives thereof, or combinations thereof.

The natural polymer of the present invention may be at least one selected from the group consisting of chitosan, alginate, and combinations thereof, but is not limited thereto.

The hydrolysis may be performed at 100 to 140°C for 50 to 100 minutes, in the step iv), and more specifically at 100 to 130°C for 50 to 90 minutes, but is not limited thereto.

The freeze-drying may be performed at a temperature of -90 to -70°C for 10 to 30 hours in the step vi), and more specifically at a temperature of -80 to -70°C for 10 to 20 hours, but is not limited thereto.

The providing PLGA microparticles according to the present invention may comprise: i-i) preparing the 1-1 solution by dissolving PLGA in a solvent; i-ii) preparing the 1-2 solution by dissolving a surfactant in distilled water; i-iii) preparing the 1-3 solution by mixing and stirring the 1-1 solution and the 1-2 solution, then removing the solvent; and i-iv) obtaining the PLGA microparticles by precipitating the PLGA microparticles from the 1-3 solution, then removing the supernatant.

The solvent may be at least one selected from the group consisting of chloroform, acetone, acetonitrile, dioxane, methoxybenzene, dichloromethane, dimethylformamide, dimethyl sulfoxide, ethyl acetate, hexafluoroisopropanol, and anisole in the step i-i), but is not limited thereto.

The surfactant may be PVA (polyvinyl alcohol) in the step i-ii), but is not limited thereto.

It may further comprise step i-v) washing the PLGA microparticles after the step i-iv).

The average size of the PLGA microparticles of the present invention may be 20 to 100 µm, and more specifically may be 20 to 70 µm.

The filler composition of the present invention may be prepared by mixing PLGA and PDO.

The providing HA and PDRN microparticles of the present invention may comprise preparing 2-1 solution by dissolving HA and PDRN microparticles in sterilized distilled water.

Furthermore, the present invention provides a filler composition for plastic surgery or chondrocyte regeneration prepared by the above preparation method.

The filler composition of the present invention may increase the secretion levels of growth factors VEGF and FGF-2, and may increase collagen activity by enhancing the expression of Collagen type I and Collagen type III.

The filler composition of the present invention comprises PLGA microparticles and HA and PDRN microparticles, and may additionally comprise PDO. The filler composition has a lower risk of side effects and higher stability than filler compositions consisting of PLLA microparticles or PCL microparticles, increases cell adhesion, has a retention period of over one year, and can promote collagen activation and skin regeneration.

Furthermore, the present invention provides an injectable formulation for plastic surgery or chondrocyte regeneration, comprising the above filler composition.

The injection formulation of the present invention can be used in a form in which it is administered to the skin area of a subject using an injection device having a fine needle or cannula shape.

The injectable formulation comprises PLGA microparticles and HA and PDRN microparticles of the present invention, and may additionally comprise a biocompatible carrier. The biocompatible carrier may comprise at least one selected from the group consisting of alginic acid and its salts, carboxymethyl cellulose and its salts, dextran and its salts, collagen, gelatin, and elastin, but is not limited thereto.

The injection formulation of the present invention may additionally comprise bioactive substances, local anesthetics, injectable water, sterile water, or distilled water depending on the application.

### ADVANTAGEOUS EFFECT

The present invention provides a filler composition for chondrocyte differentiation ability and collagen activity, and a preparation method therefor. The composition comprises a mixture of PLGA microparticles and HA and PDRN microparticles, and can be usefully used as a filler composition by stably inducing cell adsorption, increasing collagen production, activating chondrocyte regeneration, and confirming a tissue repair effect.

### BRIEF DESCRIPTION OF THE DRAWINGS

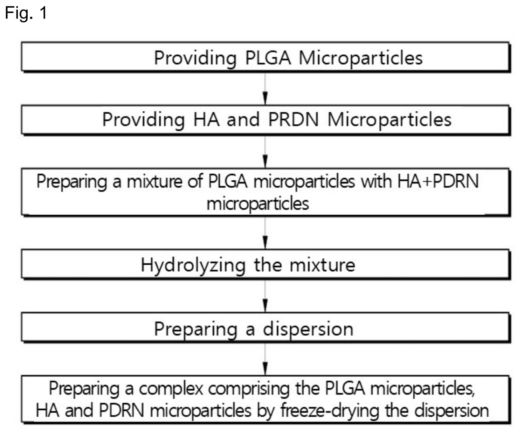

Fig. 1 is a flowchart illustrating a preparation method of a filler composition for plastic surgery and chondrocyte regeneration.
Fig. 2 is a schematic diagram illustrating the arrangement of PLGA microparticles within a filler composition for plastic surgery and chondrocyte regeneration.
Fig. 3 is a scanning electron microscope (SEM) image of the PLGA microparticles.

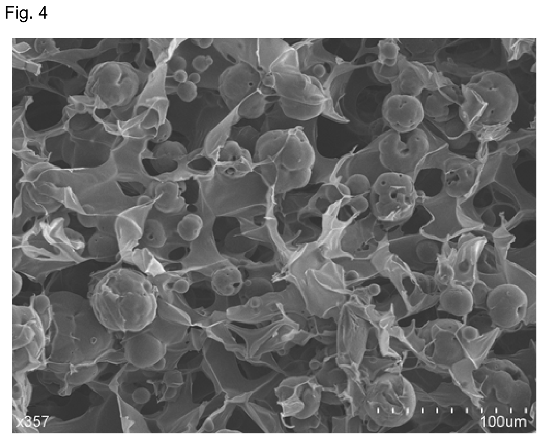

Fig. 4 is a scanning electron microscope (SEM) image of a mixture of PDRN and hyaluronic acid.
Fig. 5 is a scanning electron microscope (SEM) image of a mixture of PLGA microparticles, PDRN, and hyaluronic acid.
Fig. 6 is a diagram illustrating the secretion levels of VEGF, FGF-2, and BMP-2 for evaluating the *in vitro* efficacy of human bone marrow-derived mesenchymal stem cells cultured with a mixture comprising PLGA microparticles, PDRN, and hyaluronic acid.
Fig. 7 is a diagram showing collagen type I and III expression, chondrocyte differentiation ability, and osteogenic differentiation ability to evaluate the *in vitro* efficacy of human bone marrow-derived mesenchymal stem cells on a mixture of PLGA microparticles, PDRN, and hyaluronic acid.

### MODE FOR CARRYING OUT INVENTION

Hereinafter, the contents of the present invention will be described in more detail through the following examples and experimental examples. However, the scope of the invention is not limited to the following examples and experimental examples, but also includes modifications of technical concept equivalent thereto.

### Example 1. Preparation of PLGA Microparticles

2 g of PLGA was dissolved in 50 mL of hexafluoroisopropanol (HFIP) to prepare Solution 1-1. 2 g of PVA, as a surfactant, was dissolved in 50 mL of distilled water to prepare Solution 1-2. The resulting mixture of Solutions 1-1 and 1-2 was stirred at 400 rpm for 3 days to remove HFIP, and Solution 1-3 containing PLGA microparticles was simultaneously obtained.

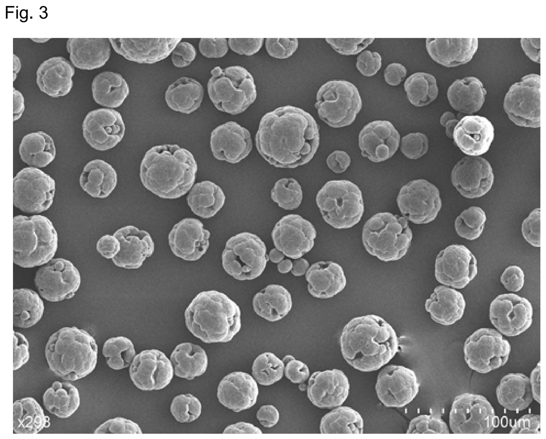

After stirring ceased, the mixture was left to stand for at least 24 hours to allow the polymer microparticles to settle. The supernatant was then removed and the PLGA microparticles were isolated. Distilled water was added to the isolated PLGA microparticles and the mixture was stirred again to wash them. This washing step was repeated three times resulting in the preparation of PLGA microparticles (Fig. 3).

### Example 2. Mixture of PLGA Microparticles with HA and PDRN Microparticles

The microparticles were mixed according to Table 1 below, and a total of five types of microparticle mixtures, each weighing 10 g, were prepared.

**Table 1**

| | PLGA | HA+PDRN |
|---|---|---|
| composition (%) | 70 | 30 |
| | 75 | 25 |
| | 80 | 20 |
| | 85 | 15 |
| | 90 | 10 |

Each type was added to 100 mL of a 15% solution containing the dispersant composition, and stirred for 2 hours to prepare a completely dispersed solution. The dispersion solution was uniformly poured into a hemispherical mold with a diameter of 1 cm or uniformly poured into a cylindrical container, and placed in an ultra-low temperature freezer at -75°C for 12 hours to ensure complete freezing.

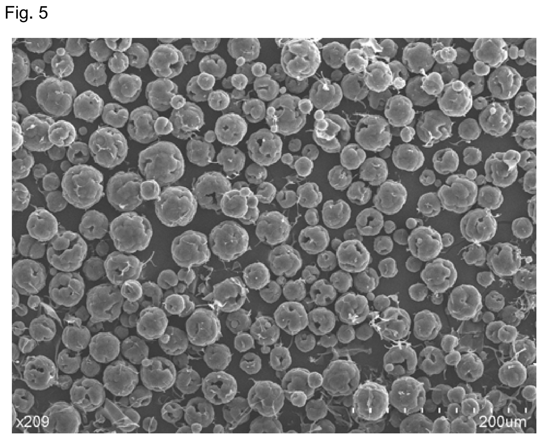

Subsequently, freeze-drying was performed to produce a completely dried form of the complex. Among the complexes, the final complex completed by mixing PLGA: HA+PDRN at a ratio of 85 : 15 is as shown in Fig. 5.

### Example 3. High-temperature, high-pressure hydrolysis treatment

The mixture of PLGA microparticles, HA + PDRN microparticles prepared in Example 2 was subjected to surface treatment using a high-temperature and high-pressure apparatus (120°C for 50 minutes), thereby improving hydrophilicity and enhancing cell adhesion through surface modification and hydrolysis of the microparticles.

### Experimental Example 1. In vitro efficacy evaluation test

A mixture of PLGA microparticles, HA + PDRN microparticles was used for in vitro testing, wherein cell markers (CD44, CD105, vimentin, and integrin β) in human bone marrow-derived mesenchymal stem cells (hMSCs) were analyzed by flow cytometry, and the secretion levels of growth factors (VEGF, FGF-2, BMP-2, PDGF-BB, TGF-β1, and IGF-1) were evaluated using ELISA.Additionally, the expression of collagen types I and III, as well as chondrocyte differentiation ability and osteogenic differentiation ability, were confirmed by immunofluorescence staining, toluidine blue staining, and alizarin red staining, respectively.

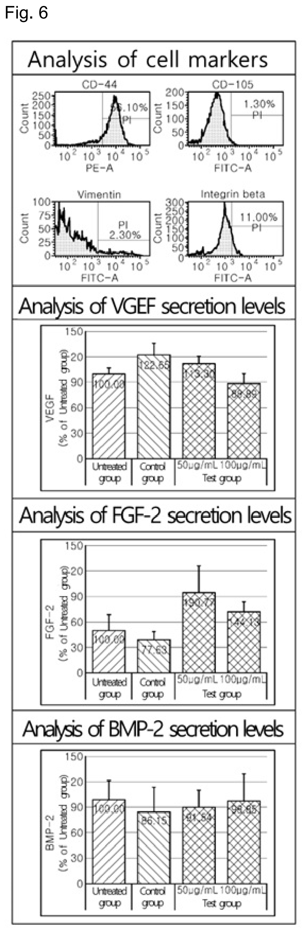

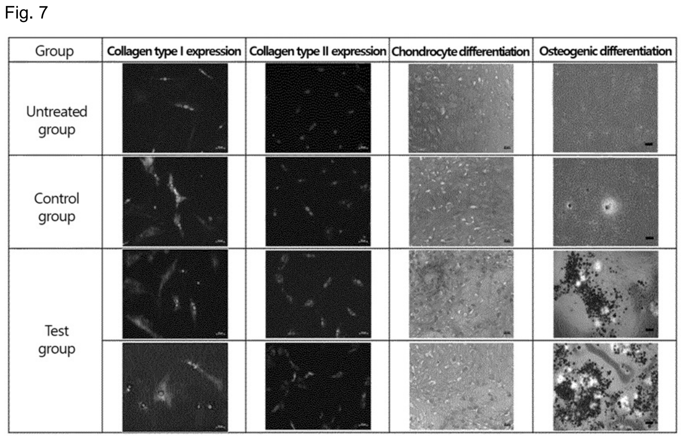

As shown in Fig. 6, when the untreated group was set as 100%, the analysis revealed that VEGF and FGF-2 levels in the test group (a mixture of PLGA, HA, and PDRN microparticles, hereafter referred to as the test group) increased at both 50 µg/mL and 100 µg/mL concentrations compared to the untreated group. The reduction in BMP-2 levels was less pronounced than that observed in the control group(Fig. 6). For collagen type I and collagen type III immunofluorescence staining, expression increased in the test groups at concentrations of 50 µg/mL and 100 µg/mL compared to the untreated group. Regarding osteogenic differentiation ability, a significant increase was observed in the test groups at concentrations of 50 µg/mL and 100 µg/mL compared to the untreated and control groups (Fig. 7).

This suggests that the mixture of PLGA microparticles with HA and PDRN microparticles is effective in improving the secretion levels of specific growth factors(VEGF and FGF-2), the expression of Collagen type I and Collagen type III, and osteogenic differentiation ability.

While the embodiments of the present invention have been described above, the present invention is not limited to the above embodiments, but can be prepared in various different forms. Those skilled in the art to which the invention pertains will understand that the present invention can be implemented in other specific forms without altering the technical concept or essential characteristics of the present invention. Therefore, the embodiments described above should be understood to be illustrative in all respects and not restrictive.

## Claims

1. A method for preparing a filler composition for plastic surgery or chondrocyte regeneration comprising the steps of:
i) preparing PLGA microparticles composed of PLGA (poly(lactic-co-glycolic acid));
ii) providing HA (hyaluronic acid) and PDRN (polydeoxyribonucleotide) microparticles;
iii) preparing a mixture by mixing the PLGA microparticles with the HA and PDRN microparticles;
iv) hydrolyzing the mixture;
v) preparing a dispersion by dispersing the hydrolyzed mixture in a solution containing a dispersant; and
vi) preparing a complex comprising the PLGA microparticles, the HA and PDRN microparticles by freeze-drying the dispersion.

2. The method for preparing a filler composition for plastic surgery or chondrocyte regeneration according to claim 1, wherein the weight ratio of the PLGA microparticles based on the total weight of the mixture is 70 to 90 wt% in the step iii).

3. The method for preparing a filler composition for plastic surgery or chondrocyte regeneration according to claim 1, wherein the weight ratio of the HA based on the total weight of the mixture is 1 to 20 wt% in the step iii).

4. The method for preparing a filler composition for plastic surgery or chondrocyte regeneration according to claim 1, wherein the weight ratio of the PDRN microparticles based on the total weight of the mixture is 0.01 to 5 wt% in the step iii).

5. The method for preparing a filler composition for plastic surgery or chondrocyte regeneration according to claim 1, wherein the dispersant is at least one selected from the group consisting of cells, growth factors, peptides, amino acids, proteins, carbohydrates, and natural polymers.

6. The method for preparing a filler composition for plastic surgery or chondrocyte regeneration according to claim 5, wherein the natural polymer is at least one selected from the group consisting of chitosan and alginate.

7. The method for preparing a filler composition for plastic surgery or chondrocyte regeneration according to claim 1, wherein the hydrolysis is performed at a temperature of 100 to 140°C for 50 to 100 minutes in the step iv).

8. The method for preparing a filler composition for plastic surgery or chondrocyte regeneration according to claim 1, wherein the freeze-drying process is performed at a temperature of -90 to -70°C for 10 to 20 hours in the step vi).

9. The method for preparing a filler composition for plastic surgery or chondrocyte regeneration according to claim 1, wherein the PLGA microparticles are provided by i-i) preparing the 1-1 solution by dissolving PLGA in a solvent; i-ii) preparing the 1-2 solution by dissolving a surfactant in distilled water; i-iii) preparing the 1-3 solution by mixing and stirring the 1-1 solution and the 1-2 solution, then removing the solvent; and i-iv) obtaining the PLGA microparticles by precipitating the PLGA microparticles from the 1-3 solution, then removing the supernatant in the step i).

10. The method for preparing a filler composition for plastic surgery or chondrocyte regeneration according to claim 9, wherein the solvent is at least one selected from the group consisting of chloroform, acetone, acetonitrile, dioxane, methoxybenzene, dichloromethane, dimethylformamide, dimethyl sulfoxide, ethyl acetate, hexafluoroisopropanol, and anisole in the step i-i).

11. The method for preparing a filler composition for plastic surgery or chondrocyte regeneration according to claim 9, wherein the surfactant is PVA (polyvinyl alcohol) in the step i-ii).

12. The method for preparing a filler composition for plastic surgery or chondrocyte regeneration according to claim 9, it further comprises step i-v) washing the PLGA microparticles after the step i-iv).

13. The method for preparing a filler composition for plastic surgery or chondrocyte regeneration according to claim 1, wherein average size of the PLGA microparticles is 20 to 100 µm.

14. A filler composition for plastic surgery or chondrocyte regeneration prepared by the method of claim 1.

15. The filler composition according to claim 14, wherein the composition increases the secretion levels of the growth factors VEGF (vascular endothelial growth factor) and FGF-2 (fibroblast growth factor-2).

16. The filler composition according to claim 14, wherein the composition increases the expression of Collagen type I and Collagen type III.

17. An injectable formulation for plastic surgery or chondrocyte regeneration, comprising the filler composition of claim 14.
